# EUROPEAN PATENT SPECIFICATION

(11) **EP 0 744 940 B1**
(45) Date of publication and mention of the grant of the patent: **11.11.1998**
(21) Application number: 95908842.8
(22) Date of filing: 14.02.1995
(51) Int. Cl.: A61K 9/16, A61K 9/50

(54) **DRUGS, VACCINES AND HORMONES IN POLYLACTIDE COATED MICROSPHERES**
ARZNEIMITTEL, IMPFSTOFFE UND HORMONE IN POLYLAKTIDBESCHICHTETEN MIKROPARTIKEL
MEDICAMENTS, VACCINS ET HORMONES INCORPORES DANS DES MICROSPHERES RECOUVERTES DE POLYLACTIDES

(30) Priority: 17.02.1994 US 197756; 17.02.1994 US 197754
(43) Date of publication of application: 04.12.1996
(73) Proprietor: MODI, Pankaj, Hamilton, Ontario L8S 1J4 (CA)
(72) Inventor: MODI, Pankaj, Hamilton, Ontario L8S 1J4 (CA)
(74) Representative: Harrison, David Christopher
(86) International application number: CA9500074
(87) International publication number: WO9522318

(56) References cited:
- WO-A-88/07870
- US-A- 4 479 911
- US-A- 4 568 559

## Description

The present invention relates to an improved delivery system for the administration of drugs, vaccines and hormones for use with animals, including humans.

### Background

It is known that many drugs and hormones, e.g. most peptidic and proteinic drugs are susceptible to degradation at the site of administration. In addition, some proteinic and peptidic drugs have very short in-vivo half lives. Consequently, multiple injections or multiple oral doses are required to achieve desirable therapy. It is desirable to increase the therapeutic efficacy of these drugs by using a controlled release delivery system. Attempts have been made to provide controlled release by means of tablets or capsules. However, none of these methods are entirely satisfactory.

Extensive efforts have been expended on the identification of appropriate antigens for immunization against numerous infectious diseases. However, the efficiency of such vaccines often is low because of rapid degradation of antigens and their very short in-vivo half lives. The need for effective vaccination procedures is particularly acute with respect to organisms which produce their pathophysiologic effects through acute infections localized to the gastrointestinal surfaces. However, large doses have been required to achieve adequate local concentrations in the peyer's patches of the gastrointestinal tract. There is a need therefore to provide a vaccine delivery system which results in enhanced immunity without the need for adjuvants and is effective following oral administration.

The present invention is intended to provide delivery system which is more cost efficient than previous methods and which alleviates the aforementioned performance difficulties. The invention is suitable for injectable and oral vaccines, but oral administration is preferred. The drug, vaccine or hormone is sometimes referred to herein as the ative ingredient. A vaccine may also be referred to herein as an antigen. WO-A-8807870 forms the body of a controlled-release microsphere from lactide-glycolide copolymer.

The present invention provides a controlled release formulation comprising biodegradable polymer microspheres wherein a drug, vaccine or hormone is suspended in a polymer matrix, said polymer matrix being formed from at least two water soluble biodegradable polymers, and said microspheres being coated with a (d,l lactide-glycolide) copolymer.

In one embodiment the polymers are selected from the group consisting of starch, crosslinked starch, ficoll, polysucrose, polyvinyl alcohol, gelatine, hydroxymethyl cellulose, hydroxyethyl cellulose, hydroxypropyl cellulose, hydroxypropyl-ethyl cellulose, hydroxypropyl-methyl cellulose, sodium carboxymethyl cellulose, cellulose acetate, sodium alginate, polymaleic anhydride esters, polyortho esters, polyethyleneimine, polyethylene glycol, methoxypolyethylene glycol, ethoxypolyethylene glycol, polyethylene oxide,poly(1,3 bis(p-carboxyphenoxy) propane-co-sebacic anhydride, N,N-diethylaminoacetate, block copolymers of polyoxyethylene and polyoxypropylene.

An example of a suitable polyortho ester is 3,9-bis(methylene)-2,4,8,10,-tetra oxaspiro[5,5]undecane/1,6 hexanediol poly (ortho ester).

It is preferred that the weight ratio of the two polymers is in the range of from 20:80 to 80:20.

In another embodiment the polymer matrix is selected from starch and ficoll, starch and polysucrose, starch and polyvinyl alcohol, starch and gelatine, starch and hydroxyethyl cellulose, hydroxyethyl cellulose and hydroxypropyl cellulose, gelatine and hydroxyethyl cellulose, gelatine and polyvinyl alcohol, polysucrose and polyvinyl alcohol, and sodium carboxymethyl cellulose and sodium alginate.

When the polymer matrix comprises starch and ficoll, the preferred weight ratio of starch to ficoll is preferably from 85:15 to 60:40, and more preferably from 75:25 to 65:35.

When the polymer matrix comprises starch and polyvinyl alcohol, the preferred weight ratio of starch to polyvinyl alcohol is in the range of from 35:65 to 65:35, with a more preferred range of from 40:60 to 60:40. A microsphere having a ratio of starch to polyvinyl alcohol of about 50:50 is suitable for release of active ingredient over about a 10 day period. The starch has a tendency to degrade relatively quickly and the polyvinyl alcohol tends to give to the microsphere hardness and makes them more resistant to enzymatic degradation. In the case of vaccines, the polyvinyl alcohol also tends to give stability toward enzymatic and proteolytic degradation. Similar ratios are suitable for polysucrose and polyvinyl alcohol.

When the polymer matrix comprises one of the celluloses and ficoll, the preferred weight ratio of the cellulose to ficoll is in the range of from 80:20 to 65:35. The celluloses, e.g. hydroxymethyl cellulose, hydroxyethyl cellulose, hydroxypropyl cellulose, hydroxypropyl-ethyl cellulose, hydroxypropyl-methyl cellulose, sodium carboxymethyl cellulose, cellulose acetate, tend to give soft and stable microspheres.

The selection of the particular (d,l lactide-glycolide) copolymer will depend in a large part on how long a period the microsphere is intended to release the active ingredient. For example, a (d,l lactide-glycolide) copolymer made from about 80% lactic acid and 20% glycolic acid is very stable and would provide an microsphere suitable for release of active ingredient over a period of weeks. A (d,l lactide-glycolide) copolymer made from 50% lactic acid and 50% glycolic acid is stable and would provide a microsphere suitable for release of active ingredient over a period of days. A (d,l lactide-glycolide) copolymer made from 20% lactic acid and 80% glycolic acid disintegrates relatively easily and would provide a microsphere suitable for release of active ingredient over a period of 1-2 days. The coating makes the microspheres more resistant to enzymatic degradation.

In another embodiment, the active ingredient is selected from the group consisting of bovine somatropine (sometimes referred to as BST), estrogens, androgens, insulin, insulin growth factors (sometimes referred to as IGF), interleukin-I, interleukin-II and cytokins. Three such cytokins are interferon-β, interferon-γ and tuftsin.

A preferred hormone, to increase the milk production in cows, is BST.

In another embodiment, the antigen is selected from the group consisting of MMR (mumps, measles and rubella) vaccine, typhoid vaccine, hepatitis A vaccine, hepatitis B vaccine, herpes simplex virus, bacterial toxoids, cholera toxin B-subunit, influenza vaccine virus, whooping cough vaccine virus, vaccinia virus, adenovirus, canary pox, polio vaccine virus, malaria vaccine virus, bacillus calmette geurin (BCG), klebsiella pneumoniae, HIV envelop glycoproteins and cytokins. Examples of bacterial toxoids are tetanus, diphtheria, pseudomonas A, mycobacterium tuberculosis. Examples of HIV envelop glycoproteins are gp 120 and gp 160 for AID vaccines.

The formulation is preferably in an oral form, although it may be in an injectable form.

The present invention also provides a process for making a controlled release formulation comprising microspheres of a drug, vaccine or hormone suspended in a biodegradable polymer matrix, said process comprising the steps of
a) preparing an aqueous solution of at least two water soluble biodegradable polymers and adding thereto an active ingredient of a hormone, vaccine or drug,
b) mixing the solution and active ingredient with an emulsifying medium to form a homogenized microdroplet suspension,
c) adding the homogenized microdroplet suspension slowly to a first organic solvent which contains a small concentration of a first surfactant, while stirring the microdroplet suspension and solvent, thus causing microspheres to precipitate,
d) separating the microspheres from the first solvent and adding a solution of a (d,l lactide-glycolide) copolymer in a second organic solvent which contains a small concentration of a second surfactant, and
e) slowly evaporating the solvent, leaving behind coated microspheres.

Step b) may be accomplished at room temperature or less but temperatures of -5°C to 10°C are preferred with a temperature in the range of from 0°C to 5°C being even more preferred. These temperature ranges are more suitable for the easy formation of the suspension.

The first organic solvent may be the same or different to the second organic solvent. A preferred first solvent is acetone and a preferred second solvent is an acetone and chloroform mixture.

Likewise the first surfactant may be the same or different to the second surfactant. Preferred surfactants are polyoxyethylene derivatives of fatty acid partial esters of sorbitol anhydrides, some of which are sold under the Tween trade mark. Preferred concentrations of surfactant are from 2 to 3% v/v of the solvent. At higher concentrations, the final microspheres tend to be irregular in shape.

In one embodiment the polymers are selected from the group consisting of starch, crosslinked starch, ficoll, polysucrose, polyvinyl alcohol, gelatine, hydroxymethyl cellulose, hydroxyethyl cellulose, hydroxypropyl cellulose, hydroxypropyl-ethyl cellulose, hydroxypropyl-methyl cellulose, sodium carboxymethyl cellulose, cellulose acetate, sodium alginate, polymaleic anhydride esters, polyortho esters, polyethyleneimine, polyethylene glycol, methoxypolyethylene glycol, ethoxypolyethylene glycol, polyethylene oxide,poly(1,3 bis(p-carboxyphenoxy) propane-co-sebacic anhydride, N,N-diethylaminoacetate, block copolymers of polyoxyethylene and polyoxypropylene.

It is preferred that the weight ratio of the two polymers is in the range of from 20:80 to 80:20, with a more preferred range of from 40:60 to 60:40.

When the polymer matrix comprises starch and ficoll, the preferred weight ratio of starch to ficoll is preferably from 85:15 to 60:40, and more preferably from 75:25 to 65:35.

When the polymer matrix comprises starch and polyvinyl alcohol, the preferred weight ratio of starch to polyvinyl alcohol is in the range of from 35:65 to 65:35, with a more preferred range of from 40:60 to 60:40. A microsphere having a ratio of starch to polyvinyl alcohol of about 50:50 is suitable for release of active ingredient over about a 10 day period. Similar ratios are suitable for polysucrose and polyvinyl alcohol.

When the polymer matrix comprises one of the celluloses and ficoll, the preferred weight ratio of the cellulose to ficoll is in the range of from 80:20 to 65:35. As indicated before, the celluloses, e.g. hydroxymethyl cellulose, hydroxyethyl cellulose, hydroxypropyl cellulose, hydroxypropyl-ethyl cellulose, hydroxypropyl-methyl cellulose, sodium carboxymethyl cellulose, cellulose acetate, tend to give soft and stable microspheres.

In another embodiment the polymer matrix is selected from starch and ficoll, starch and polysucrose, starch and polyvinyl alcohol, starch and gelatine, starch and hydroxyethyl cellulose, hydroxyethyl cellulose and hydroxypropyl cellulose, gelatine and hydroxyethyl cellulose, gelatine and polyvinyl alcohol, polysucrose and polyvinyl alcohol, and sodium carboxymethyl cellulose and sodium alginate.

In another embodiment, the active ingredient is selected from bovine somatropine (sometimes referred to as BST), estrogens, androgens, insulin, insulin growth factors (sometimes referred to as IGF), interleukin-I, interleukin-II and cytokins. Three such cytokins are interferon-β, interferon-γ and tuftsin.

A preferred hormone, to increase the milk production in cows, is BST.

In another embodiment, the antigen is selected from MMR (mumps, measles and rubella) vaccine, typhoid vaccine, hepatitis A vaccine, hepatitis B vaccine, herpes simplex virus, bacterial toxoids, cholera toxin B-subunit, influenza vaccine virus, whooping cough vaccine virus, vaccinia virus, adenovirus, canary pox, polio vaccine virus, malaria vaccine virus, bacillus calmette geurin (BCG), klebsiella pneumoniae, HIV envelop glycoproteins and cytokins. Examples of bacterial toxoids are tetanus, diphtheria, pseudomonas A, mycobacterium tuberculosis. Examples of HIV envelop glycoproteins are gp 120 and gp 160 for AID vaccines.

In the first step of the process, the polymers which are intended to be used for the matrix are added to water, to form an aqueous solution, and the drug, vaccine or hormone is added to the solution. This mixture is stirred and then mixed with an emulsifying medium, for example corn oil, rapeseed oil, vegetable oil. It is preferred that the amount of emulsifying medium exceed the amount of the polymer solution. For example, there may be as much as four times the amount of emulsifying medium as there is polymer solution. As indicated above, it is preferable that the mixture and emulsifying medium are held at a temperature lower than room temperature, and preferably between -5°C and 10°C, especially from about 0°C to about 5°C. The polymer, water, emulsifying medium and drug, vaccine or hormone are stirred or otherwise mixed until a coarse preemulsion is formed. The pre-emulsion is further homogenized to form a microdroplet suspension, for example by high speed mixing. In the laboratory, such high speed mixing can be accomplished by sonication. In commercial manufacture, a high speed blender or turbine mixer would be preferred. In the third step, the microdroplet suspension preferably is poured slowly into a large amount of organic solvent such as acetone, containing a small concentration of surfactant, e.g. about 2% v/v Tween® 80 surfactant, while stirring at high speed. Tween surfactants are available from Atlas Chemical Industries in the U.S.A. Microspheres of the drug, vaccine or hormone in the polymer matrix precipitate. The purpose of the surfactant is to prevent agglomeration of the polymer matrix, with consequent loss of microsphere-sized particles.

The particle size of the precipitate is in the order of 100 nm to 100 µm, more typically in the 10 nm to 10 µm range.

In the fourth step, the microspheres are further coated with a (d,l lactide-glycolide) copolymer by transferring the microspheres into a solution of the copolymer in a second organic solvent which contains a second surfactant. For example, the microspheres may be added to a 2% solution of (d,l lactide-glycolide) copolymer in a 1:1 acetone/chloroform mixture which contains 1% v/v Tween® 80.

In the last step the second solvent and any remaining first solvent is removed by evaporation, so that a coating of (d,l lactide-glycolide) copolymer is left on the surface of the microspheres. A mild vacuum may be used to assist in the evaporation process.

The microspheres may then be made up in oral form or injectable form. The oral form is preferred. Clearly, the amounts and type of drug or hormone will depend on the treatment or immunization required and the human being or animal being treated.

For hormones and drugs, an advantage of the microencapsulation is that it increases the in-vivo half lives of many peptidic and proteinic drugs or hormones, by providing protection against proteolytic or enzymatic degradation. Another advantage is that the therapeutic efficacies may be increased as a result of releasing the active ingredient in a controlled fashion over a prolonged period of time. A further advantage is prevention of degradation of the active ingredients when, in oral form, they pass through the gastrointestinal tract.

For vaccines, an advantage of the microencapsulation is that it increases the potency of weak antigens such as the small synthetic or recombinant peptides of HIV. Another advantage is that it enhances the speed, rigor and persistence of the immune response. A further advantage is that it modulates antibody avidity, specificity, quantity, isotype and subclass. Furthermore it decreases the amount of antigen needed to protect, thereby decreasing the cost and increasing the bioavailablility of the vaccines. Additionally, the microspherical form of the vaccine is more efficacious than an aqueous vaccine.

Microsphere formulations of the present invention which include BST may be used to increase milk production in cattle. Usually the formulation will be mixed with cattle feed and therefore will be taken orally. Such compositions may give high blood levels of BST over a long period of time, for example about 30 days, as a result of sustained release of natural or recombinant BST.

The present invention may be used to entrap other growth hormones in a polymer matrix, e.g. estrogens, androgens, insulin, IGF, interleukin-I and interleukin-II. Cytokins such as interferon-β and interferon-γ, used in the treatment of diseases such as osteoporosis, diabetes mellitus and multiple sclerosis may also benefit from the present invention.

It will be understood by those skilled in the art that all of the ingredients must be suitable for ingestion by the animal, e.g. human. This means of course that toxic ingredients falling within the literal scope of the named chemical families mentioned herein would not be chosen.

The invention is illustrated by the following non-limiting examples.

### Example I

0.1 g starch was dissolved in 1 ml of dimethylsulphoxide (DMSO) and to this solution was added 0.5 g ethylhydroxycellulose and 0.1 g myoglobin protein dissolved in 1 ml water. The solution was stirred to make a homogenous solution. To this solution was added 10 ml vegetable oil at 0°C and the resulting mixture was sonicated to form a microdroplet suspension. The microdroplet suspension was added slowly to 200 ml acetone containing 2% v/v Tween® 80 surfactant. Microspheres were precipitated from the acetone. The acetone was decanted off and the microspheres were dried.

A 0.1 g sample of the microspheres was taken and the microspheres suspended in 3 ml of distilled water. This suspension was transferred to a UV cuvette. The absorbance of the myoglobin protein at 280 nm wavelength was observed over a period of 120 days and the percent of release of the myoglobin calculated. The results are shown in Table I.

**Table I**

| Time (Days) | % Myoglobin Released |
|---|---|
| 5 | 11 |
| 10 | 23 |
| 30 | 32 |
| 60 | 45 |
| 90 | 59 |
| 120 | 86 |

This shows the slow release of the myoglobin over a long period of time.

### Example II

The experiment of Example I was repeated except the myoglobin protein was replaced with fluorescenated human serum albumine (FHSA). Although not normally used in animals, FHSA was used in this experiment to show the release characteristics. Animal drugs and hormones are expected to act in a similar manner. The absorbance of FHSA was monitored at 390 nm wavelength over a period of 90 days and the percent of FHSA released was calculated. The results are shown in Table II

**Table II**

| Time (Days) | % FHSA Released |
|---|---|
| 1 | 9 |
| 2 | 11 |
| 3 | 20 |
| 7 | 23 |
| 11 | 31 |
| 30 | 38 |
| 60 | 54 |
| 90 | 92 |

This shows the slow and almost complete release of the FHSA.

### Example III

0.5 g starch was dissolved in 1 ml of dimethylsulphoxide (DMSO) and to this solution was added 0.5 g ethylhydroxycellulose and 0.1 g fluorescenated human serum albumine (FHSA) dissolved in 1 ml water. The solution was stirred to make a homogenous solution. To this solution was added 10 ml vegetable oil at 0°C and the resulting mixture was sonicated to form a microdroplet suspension. The microdroplet suspension was added slowly to a 200 ml acetone containing 2% v/v Tween® 80 surfactant. Microspheres were precipitated from the acetone. The acetone was decanted off and the microspheres were dried.

A 0.1 g sample of the microspheres was taken and the microspheres suspend in 3 ml of distilled water. This suspension was transferred to a UV cuvette. The absorbance of the FHSA at 390 nm wavelength was observed over a period of 90 days. The absorbance is indicative of the amount of FHSA released. The results are shown in Table III.

**Table III**

| Time (Days) | Absorbance |
|---|---|
| 1 | 0.18 |
| 2 | 0.21 |
| 3 | 0.40 |
| 7 | 0.45 |
| 11 | 0.61 |
| 30 | 0.77 |
| 60 | 1.10 |
| 90 | 1.85 |

Table III shows the slow release of the FHSA.

### Example IV

The experiment of Example III was repeated except the flourescenated human serum albumine (FHSA) was replaced with myoglobin protein. The absorbance of myoglobin protein was monitored at 280 nm wavelength over a period of 120 days. The results are shown in Table IV

**Table IV**

| Time (Days) | % Myoglobin Released |
|---|---|
| 5 | 11 |
| 10 | 24 |
| 30 | 32 |
| 60 | 45 |
| 90 | 59 |
| 120 | 86 |

This shows the slow and steady release of the myoglobin protein.

### Example V

Human serum albumine (HSA) microspheres were made as in Example III except that the HSA was not fluorescenated. 50 mg of the microspheres were gavaged orally to Balb-C mice. The mice were bled at regular intervals and the blood samples centrifuged to isolate the plasma. The plasma was analyzed using ELISA techniques to estimate antibody titre (IgG)values. The results are shown in Table V.

**Table V**

| Time (Days) | Antibody titre (IgG) (Thousands) |
|---|---|
| 30 | 4 |
| 65 | 24 |
| 93 | 96 |

### Example VI

Human serum albumine (HSA) microspheres were made as in Example III except that the HSA was not fluorescenated and the hydroxyethyl cellulose was replaced by polyvinyl alcohol. 50 mg of the microspheres were gavaged orally to Balb-C mice. The mice were bled at regular intervals and the blood samples centrifuged to isolate the plasma. The plasma was analyzed using ELISA techniques to estimate antibody titre (IgG)values. The results are shown in Table IV.

**Table VI**

| Time (Days) | Antibody titre (IgG) (Thousands) |
|---|---|
| 30 | 6 |
| 65 | 13 |
| 93 | 98 |

### Example VII

0.1 g of starch was dissolved in 0.2 ml dimethylsulphoxide (DMSO) and to this solution was added 0.5 g ethylhydroxycellulose and 0.051 g bromophenol blue dye dissolved in 1 ml water. The solution was stirred to make a homogenous solution. To this solution was added 10 ml vegetable oil at 0°C and the resulting mixture was sonicated to form a microdroplet suspension. The microdroplet suspension was added slowly to 200 ml acetone containing 2% v/v Tween® 80 surfactant. Microspheres were precipitated from the acetone. The acetone was decanted off and the microspheres were dried.

The microspheres were further coated with a poly (lactide-co-glycolide) copolymer (75:25 lactide :glycolide ratio), having a molecular weight of about 80 000. The coating was applied by transferring the microspheres into a 2% solution of the poly (lactide-co-glycolide) copolymer in a 1:1 acetone/chloroform mixture which contained 1% v/v Tween® 80. The acetone and chloroform was removed by evaporation, so that a coating of (d,l lactide-glycolide) copolymer was left on the surface of the microspheres. A mild vacuum was used to assist in the evaporation process.

0.1 g of the coated microspheres was suspended in 3 ml water and evaluated for release characteristics, as shown in Table VII, overleaf.

**Table VII**

| In vitro release of bromphenol dye: | |
|---|---|
| Time (days) | % Release |
| 1 | 15.8 |
| 2 | 20.1 |
| 5 | 25.0 |
| 10 | 35.0 |
| 30 | 38.0 |
| 40 | 47.5 |
| 60 | 50.0 |

This experiment shows that the coating prevented premature degradation of the microspheres in an in vivo condition. Thus it illustrates that thus-coated microspheres will provide sustained release of peptides/proteins.

## Claims

1. A controlled release formulation comprising biodegradable polymer microspheres wherein a drug, vaccine or hormone is suspended in a polymer matrix, said polymer matrix being formed from at least two water soluble biodegradable polymers, and said microspheres being coated with a (d,l lactide-glycolide) copolymer.

2. A formulation according to Claim 1 wherein the polymers are selected from the group consisting of starch, crosslinked starch, ficoll, polysucrose, polyvinyl alcohol, gelatine, hydroxymethyl cellulose, hydroxyethyl cellulose, hydroxypropyl cellulose, hydroxypropyl-ethyl cellulose, hydroxypropyl-methyl cellulose, sodium carboxymethyl cellulose, cellulose acetate, sodium alginate, polymaleic anhydride esters, polyortho esters, polyethyleneimine, polyethylene glycol, methoxypolyethylene glycol, ethoxypolyethylene glycol, polyethylene oxide,poly(1,3 bis(p-carboxyphenoxy) propane-co-sebacic anhydride, N,N-diethylaminoacetate, block copolymers of polyoxyethylene and polyoxypropylene.

3. A formulation according to Claim 1 wherein the weight ratio of the two polymers is in the range of from 20:80 to 80:20.

4. A formulation according to Claim 3 wherein the polymer matrix is selected from starch and ficoll, starch and polysucrose, starch and polyvinyl alcohol, starch and gelatine, starch and hydroxyethyl cellulose, hydroxyethyl cellulose and hydroxypropyl cellulose, gelatine and hydroxyethyl cellulose, gelatine and polyvinyl alcohol, polysucrose and polyvinyl alcohol, and sodium carboxymethyl cellulose and sodium alginate.

5. A formulation according to Claim 4 wherein the polymer matrix comprises starch and ficoll and the weight ratio of starch to ficoll is from 85:15 to 60:40.

6. A formulation according to Claim 5 wherein the weight ratio is from 75:25 to 65:35.

7. A formulation according to Claim 4 wherein the polymer matrix comprises starch and polyvinyl alcohol and the weight ratio of starch to polyvinyl alcohol is in the range of from 35:65 to 65:35.

8. A formulation according to Claim 7 wherein the weight ratio is from 40:60 to 60:40.

9. A formulation according to Claim 4 wherein the polymer matrix comprises polysucrose and polyvinyl alcohol and the weight ratio of polysucrose to polyvinyl alcohol is from 35:65 to 65:35.

10. A formulation according to Claim 4 wherein the polymer matrix comprises ficoll and a cellulose selected from the group consisting of hydroxymethyl cellulose, hydroxyethyl cellulose, hydroxypropyl cellulose, hydroxypropyl-ethyl cellulose, hydroxypropyl-methyl cellulose, sodium carboxymethyl cellulose and cellulose acetate and the weight ratio of the cellulose to ficoll is in the range of from 80:20 to 65:35.

11. A formulation according to Claim 1 or 2 wherein the active ingredient is selected from the group consisting of bovine somatropine, estrogens, androgens, insulin, insulin growth factors, interleukin-I, interleukin-II and cytokins.

12. A formulation according to Claim 1 or 2 wherein the active ingredient is selected from the group consisting of MMR (mumps, measles and rubella) vaccine, typhoid vaccine, hepatitis A vaccine, hepatitis B vaccine, herpes simplex virus, bacterial toxoids, cholera toxin B-subunit, influenza vaccine virus, whooping cough vaccine virus, vaccinia virus, adenovirus, canary pox, polio vaccine virus, malaria vaccine virus, bacillus calmette geurin, klebsiella pneumoniae, and HIV envelop glycoproteins.

13. A process for making a controlled release formulation comprising microspheres of a drug, vaccine or hormone suspended in a biodegradable polymer matrix polymer, said process comprising the steps of
a) preparing an aqueous solution of at least two water soluble biodegradable polymers and adding thereto an active ingredient of a hormone, vaccine or drug,
b) mixing the solution and active ingredient with an emulsifying medium to form a homogenized microdroplet suspension,
c) adding the homogenized microdroplet suspension slowly to a first organic solvent which contains a small concentration of a first surfactant, while stirring the microdroplet suspension and solvent, thus causing microspheres to precipitate,
d) separating the microspheres from the first solvent and adding a solution of a (d,l lactide-glycolide) copolymer in a second organic solvent which contains a small concentration of a second surfactant, and
e) slowly evaporating the solvent, leaving behind coated microspheres.

14. A process according to Claim 13 wherein the polymers used in step a) are selected from the group consisting of starch, crosslinked starch, ficoll, polysucrose, polyvinyl alcohol, gelatine, hydroxymethyl cellulose, hydroxyethyl cellulose, hydroxypropyl cellulose, hydroxypropyl-ethyl cellulose, hydroxypropyl-methyl cellulose, sodium carboxymethyl cellulose, cellulose acetate, sodium alginate, polymaleic anhydride esters, polyortho esters, polyethyleneimine, polyethylene glycol, methoxypolyethylene glycol, ethoxypolyethylene glycol, polyethylene oxide,poly(1,3 bis(p-carboxyphenoxy) propane-co-sebacic anhydride, N,N-diethylaminoacetate, block copolymers of polyoxyethylene and polyoxypropylene.

15. A process according to Claim 13 wherein the weight ratio of the two polymers used in step a) is in the range of from 20:80 to 80:20.

16. A process according to Claim 15 wherein the polymer matrix is selected from starch and ficoll, starch and polysucrose, starch and polyvinyl alcohol, starch and gelatine, starch and hydroxyethyl cellulose, hydroxyethyl cellulose and hydroxypropyl cellulose, gelatine and hydroxyethyl cellulose, gelatine and polyvinyl alcohol, polysucrose and polyvinyl alcohol, and sodium carboxymethyl cellulose and sodium alginate.

17. A process according to Claim 16 wherein the polymer matrix comprises starch and ficoll and the weight ratio of starch to ficoll is from 85:15 to 60:40.

18. A process according to Claim 17 wherein the weight ratio is from 75:25 to 65:35.

19. A process according to Claim 16 wherein the polymer matrix comprises starch and polyvinyl alcohol and the weight ratio of starch to polyvinyl alcohol is in the range of from 35:65 to 65:35.

20. A process according to Claim 19 wherein the weight ratio is from 40:60 to 60:40.

21. A process according to Claim 16 wherein the polymer matrix comprises polysucrose and polyvinyl alcohol and the weight ratios of polysucrose to polyvinyl alcohol is from 35:65 to 65:35.

22. A process according to Claim 16 wherein the polymer matrix comprises ficoll and a cellulose selected from the group consisting of hydroxymethyl cellulose, hydroxyethyl cellulose, hydroxypropyl cellulose, hydroxypropyl-ethyl cellulose, hydroxypropyl-methyl cellulose, sodium carboxymethyl cellulose and cellulose acetate and the weight ratio of the cellulose to ficoll is in the range of from 80:20 to 65:35.

23. A process according to Claim 13 or 14 wherein the active ingredient is selected from bovine somatropine, estrogens, androgens, insulin, insulin growth factors, interleukin-I, interleukin-II and cytokins.

24. A process according to Claim 13 or 14 wherein the active ingredient is selected from MMR (mumps, measles and rubella) vaccine, typhoid vaccine, hepatitis A vaccine, hepatitis B vaccine, herpes simplex virus, bacterial toxoids, cholera toxin B-subunit, influenza vaccine virus, whooping cough vaccine virus, vaccinia virus, adenovirus, canary pox, polio vaccine virus, malaria vaccine virus, bacillus calmette geurin, klebsiella pneumoniae, and HIV envelop glycoproteins.

25. A method for increasing milk production of a cow comprising administering as the hormone in a controlled release formulation according to claim 1 or claim 2.

## Patentansprüche

1. Formulierung zur gesteuerten Freisetzung, umfassend biologisch abbaubare Polymer-Mikrokügelchen, worin ein Arzneimittel, Impfstoff oder Hormon in einer Polymermatrix suspendiert ist, die aus zumindest zwei wasserlöslichen biologisch abbaubaren Polymeren besteht, wobei die Mikrokügelchen mit einem d,l-Lactid-Glykolid-Copolymer beschichtet sind.

2. Formulierung nach Anspruch 1, worin die Polymere aus der Gruppe bestehend aus Stärke, vernetzter Stärke, Ficoll, Polysaccharose, Polyvinylalkohol, Gelatine, Hydroxymethylcellulose, Hydroxyethylcellulose, Hydroxypropylcellulose, Hydroxypropylethylcellulose, Hydroxypropylmethylcellulose, Natriumcarboxymethylcellulose, Celluloseacetat, Natriumalginat, Polymaleinsäureanhydridester, Polyorthoester, Polyethylenimin, Polyethylenglykol, Methoxypolyethylenglykol, Ethoxypolyethylenglykol, Polyethylenoxid, Poly(1,3-bis-(p-carboxyphenoxy)propan-co-sebacinsäureanhyrid), N,N-Diethylaminacetat, Blockcopolymere von Polyoxyethylen mit Polyoxypropylen, ausgewählt sind.

3. Formulierung nach Anspruch 1, worin das Gewichtsverhältnis der beiden Polymere im Bereich von 20:80 bis 80:20 liegt.

4. Formulierung nach Anspruch 3, worin die Polymermatrix aus Stärke und Ficoll, Stärke und Polysaccharose, Stärke und Polyvinylalkohol, Stärke und Gelatine, Stärke und Hydroxyethylcellulose, Hydroxyethylcellulose und Hydroxypropylcellulose, Gelatine und Hydroxyethylcellulose, Gelatine und Polyvinylalkohol, Polysaccharose und Polyvinylalkohol sowie Natriumcarboxymethylcellulose und Natriumalginat ausgewählt ist.

5. Formulierung nach Anspruch 4, worin die Polymermatrix Stärke und Ficoll umfaßt und das Gewichtsverhältnis zwischen Stärke und Ficoll von 85:15 bis 60:40 reicht.

6. Formulierung nach Anspruch 5, worin das Gewichtsverhältnis von 75:25 bis 65:35 reicht.

7. Formulierung nach Anspruch 4, worin die Polymermatrix Stärke und Polyvinvlalkohol umfaßt und das Gewichtsverhältnis zwischen Stärke und Polyvinylalkohol im Bereich von 35:65 bis 65:35 liegt.

8. Formulierung nach Anspruch 7, worin das Gewichtsverhältnis von 40:60 bis 60:40 reicht.

9. Formulierung nach Anspruch 4, worin die Polymermatrix Polysaccharose und Polyvinylalkohol umfaßt und das Gewichtsverhältnis zwischen Polysaccharose und Polyvinylalkohol von 35:65 bis 65:35 reicht.

10. Formulierung nach Anspruch 4, worin die Polymermatrix Ficoll und eine Cellulose umfaßt, die aus der Gruppe bestehend aus Hydroxymethylcellulose, Hydroxyethylcellulose, Hydroxypropylcellulose, Hydroxypropylethylcellulose, Hydroxypropylmethylcellulose, Natriumcarboxymethylcellulose und Celluloseacetat ausgewählt ist, und das Gewichtsverhältnis zwischen Cellulose und Ficoll im Bereich von 80:20 bis 65:35 liegt.

11. Formulierung nach Anspruch 1 oder 2, worin der Wirkstoff aus der Gruppe bestehend aus Rinder-Somatropin, Östrogenen, Androgenen, Insulin, Insulin-Wachstumsfaktoren, Interleukin-I, Interleukin-II und Cytokinen ausgewählt ist.

12. Formulierung nach Anspruch 1 oder 2, worin der Wirkstoff aus der Gruppe bestehend aus Impfstoffen gegen MMR (Mumps, Masern und Röteln), Typhus, Hepatitis A, Hepatitis B und Herpes simplex Virus, bakteriellen Toxoiden, Cholera-Toxin-Untereinheit B, Impfstoffen gegen Grippevirus, Keuchhustenvirus, Kuhpockenvirus, Adenovirus, Windpocken, Poliovirus, Malariavirus, Bacillus Calmette Guérin und Klebsiella pneumoniae sowie HIV-Hüllenglykoproteinen ausgewählt ist.

13. Verfahren zur Herstellung einer Formulierung zur gesteuerten Freisetzung, umfassend Mikrokügelchen eines Arzneimittels, Impfstoffs oder Hormons, suspendiert in einem Polymer einer biologisch abbaubaren Polymermatrix, wobei das Verfahren die folgenden Schritte umfaßt:
a) Bilden einer wäßrigen Lösung von zumindest zwei wasserlöslichen biologisch abbaubaren Polymeren und Hinzufügen eines Wirkstoffs eines Hormons, Impfstoffs oder Arzneimittels;
b) Vermischen der Lösung und des Wirkstoffs mit einem Emulgatormedium, um eine homogenisierte Mikrotröpfchen-Suspension zu bilden;
c) langsame Zugabe der homogenisierten Mikrotröpfchen-Suspension zu einem ersten organischen Lösungsmittel, das eine geringe Konzentration eines ersten Tensids enthält, während die Mikrotröpfchen-Suspension und das Lösungsmittel gerührt werden, wodurch Fällung der Mikrokügelchen bewirkt wird;
d) Abtrennen der Mikrokügelchen vom ersten Lösungsmittel und Zugabe einer Lösung eines d,l-Lactid-Glykolid-Copolymers in einem zweiten organischen Lösungsmittel, das eine geringe Konzentration eines zweiten Tensids enthält; und
e) langsames Verdampfen des Lösungsmittels, wodurch beschichtete Mikrokügelchen zurückbleiben.

14. Verfahren nach Anspruch 13, worin die Polymere in Schritt a) aus der Gruppe bestehend aus Stärke, vernetzter Stärke, Ficoll, Polysaccharose, Polyvinylalkohol, Gelatine, Hydroxymethylcellulose, Hydroxyethylcellulose, Hydroxypropylcellulose, Hydroxypropylethylcellulose, Hydroxypropylmethylcellulose, Natriumcarboxymethylcellulose, Celluloseacetat, Natriumalginat, Polymaleinsäureanhydridester, Polyorthoester, Polyethylenimin, Polyethylenglykol, Methoxypolyethylenglykol, Ethoxypolyethylenglykol, Polyethylenoxid, Poly(1,3-bis-(p-carboxyphenoxy)propan-co-sebacinsäureanhyrid), N,N-Diethylaminacetat, Blockcopolymere von Polyoxyethylen mit Polyoxypropylen, ausgewählt sind.

15. Verfahren nach Anspruch 13, worin das Gewichtsverhältnis der beiden Polymere im Bereich von 20:80 bis 80:20 liegt.

16. Verfahren nach Anspruch 15, worin die Polymermatrix aus Stärke und Ficoll, Stärke und Polysaccharose, Stärke und Polyvinylalkohol, Stärke und Gelatine, Stärke und Hydroxyethylcellulose, Hydroxyethylcellulose und Hydroxypropylcellulose, Gelatine und Hydroxyethylcellulose, Gelatine und Polyvinylalkohol, Polysaccharose und Polyvinylalkohol sowie Natriumcarboxymethylcellulose und Natriumalginat ausgewählt ist.

17. Verfahren nach Anspruch 16, worin die Polymermatrix Stärke und Ficoll umfaßt und das Gewichtsverhältnis zwischen Stärke und Ficoll von 85:15 bis 60:40 reicht.

18. Verfahren nach Anspruch 17, worin das Gewichtsverhältnis von 75:25 bis 65:35 reicht.

19. Verfahren nach Anspruch 16, worin die Polymermatrix Stärke und Polyvinylalkohol umfaßt und das Gewichtsverhältnis zwischen Stärke und Polyvinylalkohol im Bereich von 35:65 bis 65:35 liegt.

20. Verfahren nach Anspruch 19, worin das Gewichtsverhältnis von 40:60 bis 60:40 reicht.

21. Verfahren nach Anspruch 16, worin die Polymermatrix Polysaccharose und Polyvinylalkohol umfaßt und das Gewichtsverhältnis zwischen Polysaccharose und Polyvinylalkohol 35:65 bis 65:35 beträgt.

22. Verfahren nach Anspruch 16, worin die Polymermatrix Ficoll und eine Cellulose umfaßt, die aus der Gruppe bestehend aus Hydroxymethylcellulose, Hydroxyethylcellulose, Hydroxypropylcellulose, Hydroxypropylethylcellulose, Hydroxypropylmethylcellulose, Natriumcarboxymethylcellulose und Celluloseacetat ausgewählt ist und das Gewichtsverhältnis zwischen der Cellulose und Ficoll im Bereich von 80:20 bis 65:35 liegt.

23. Verfahren nach Anspruch 13 oder 14, worin der Wirkstoff aus Rinder-Somatropin, Östrogenen, Androgenen, Insulin, Insulin-Wachstumsfaktoren, Interleukin-I, Interleukin-II und Cytokinen ausgewählt ist.

24. Verfahren nach Anspruch 13 oder 14, worin der Wirkstoff aus Impfstoffen gegen MMR (Mumps, Masern und Röteln), Typhus, Hepatitis A, Hepatitis B und Herpes simplex Virus, bakteriellen Toxoiden, Cholera-Toxin-Untereinheit B, Impfstoffen gegen Grippevirus, Keuchhustenvirus, Kuhpockenvirus, Adenovirus, Windpocken, Poliovirus, Malariavirus, Bacillus Calmette Guérin und Klebsiella pneumoniae sowie HIV-Hüllenglykoproteinen ausgewählt ist.

25. Verfahren zur Steigerung der Milchproduktion einer Kuh, umfassend die Verabreichung von BST als Hormon, in einer Formulierung zur gesteuerten Freisetzung nach Anspruch 1 oder 2.

## Revendications

1. Formulation à libération contrôlée comprenant des microsphères de polymère biodégradable où un médicament, un vaccin ou une hormone est mis en suspension dans une matrice polymérique, ladite matrice polymérique étant formée à partir d'au moins deux polymères biodégradables solubles dans l'eau, et lesdites microsphères étant revêtues d'un copolymère de (d,l lactide-glycolide).

2. Formulation selon la revendication 1 dans laquelle les polymères sont sélectionnés dans le groupe consistant en l'amidon, l'amidon réticulé, le ficoll, le polysucrose, l'alcool polyvinylique, l'hydroxyméthyl cellulose, l'hydroxyéthyl cellulose, l'hydroxypropyl cellulose, l'hydroxypropyl-éthyl cellulose, l'hydroxypropyl-méthyl cellulose, la sodium carboxyméthyl cellulose, la cellulose acétate, le sodium alginate, les esters d'anhydride polymaléïque, les polyortho esters, la polyéthyléneimine, le polyéthylène glycol, le méthoxypolyéthylène glycol, l'éthoxypolyéthylène glycol, le polyéthylène oxyde, l'anhydride poly(1,3 bis(p-carboxyphénoxy)propane-co-sébacique, le N,N-diéthylaminoacétate, les copolymères blocs de polyoxyéthylène et de polyoxypropylène.

3. Formulation selon la revendication 1 dans laquelle le rapport en poids des deux polymères est dans l'intervalle de 20:80 à 80:20.

4. Formulation selon la revendication 3 dans laquelle la matrice polymérique est sélectionnée parmi l'amidon et le ficoll, l'amidon et le polysucrose, l'amidon et l'alcool polyvinylique, l'amidon et la gélatine, l'amidon et l'hydroxyéthyl cellulose, l'hydroxyéthyl cellulose et l'hydroxypropyl cellulose, la gélatine et l'hydroxyéthyl cellulose, la gélatine et l'alcool polyvinylique, le polysucrose et l'alcool polyvinylique, et la sodium carboxyméthyl cellulose et le sodium alginate.

5. Formulation selon la revendication 4, dans laquelle la matrice polymérique comprend de l'amidon et du ficoll et le rapport en poids de l'amidon au ficoll est de 85:15 à 60:40.

6. Formulation selon la revendication 5, dans laquelle le rapport en poids est de 75:25 à 65:35.

7. Formulation selon la revendication 4 dans laquelle la matrice polymérique comprend de l'amidon et de l'alcool polyvinylique et le rapport en poids de l'amidon à l'alcool polyvinylique est dans l'intervalle de 35:65 à 65:35.

8. Formulation selon la revendication 7 dans laquelle le rapport en poids est de 40:60 à 60:40.

9. Formulation selon la revendication 4 dans laquelle la matrice polymérique comprend le polysucrose et l'alcool polyvinylique et le rapport en poids du polysucrose à l'alcool polyvinylique est de 35:65 à 65:35.

10. Formulation selon la revendication 4 dans laquelle la matrice polymérique comprend du ficoll et une cellulose sélectionnée dans le groupe consistant en l'hydroxyméthyl cellulose, l'hydroxéthyl cellulose, l'hydroxypropyl cellulose, l'hydroxypropyl-éthyl cellulose, l'hydroxypropyl-méthyl cellulose, la sodium carboxyméthyl cellulose et l'acétate de cellulose et le rapport en poids de la cellulose au ficoll est dans l'intervalle de 80:20 à 65:35.

11. Formulation selon la revendication 1 ou 2, dans laquelle l'ingrédient actif est sélectionné dans le groupe consistant en la somatropine bovine, les oestrogènes, les androgènes, l'insuline, les facteurs de croissance d'insuline, l'interleukine-I, l'interleukine-II et les cytokines.

12. Formulation selon la revendication 1 ou 2, dans laquelle l'ingrédient actif est sélectionné dans le groupe consistant en le vaccin MMR (oreillons, rougeole et rubéole), le vaccin pour la typhoïde, le vaccin contre l'hépatite A, le vaccin contre l'hépatite B, le virus herpès simplex, les toxoïdes bactériens, la sous-unité B de la toxine du choléra, le virus du vaccin de la grippe, le virus du vaccin de la coqueluche, le virus de la vaccine, un adénovirus, la variole du canari, le virus du vaccin de la polio, le virus du vaccin de la malaria, le bacillus calmette geurin, le klebsiella pneumoniae, et des glycoprotéines d'enveloppe de VIH.

13. Procédé pour fabriquer une formulation à libération contrôlée comprenant des microsphères d'un médicament, d'un vaccin ou d'une hormone mises en suspension dans un polymère de matrice polymérique biodégradable, ledit procédé comprenant les étapes de
a) préparation d'une solution aqueuse d'au moins deux polymères biodégradables solubles dans l'eau et ajout à ceux-ci d'un ingrédient actif d'une hormone, d'un vaccin ou d'un médicament,
b) mélange de la solution et l'ingrédient actif avec un milieu émulsifiant pour former une suspension de microgoutellettes homogénéisées,
c) ajout de la suspension homogénéisée des microgoutellettes lentement dans un solvant organique qui contient une petite concentration d'un premier surfactant, tout en agitant la suspension de microgoutellettes et le solvant, faisant ainsi précipiter les microsphères,
d) séparation des microsphères du premier solvant et ajout d'une solution d'un copolymère de (d,l lactide-glycolide) dans un second solvant organique qui contient une petite concentration d'un second surfactant, et
e) évaporation lentement du solvant, laissant derrière des microsphères revêtues.

14. Procédé selon la revendication 13 dans lequel les polymères utilisés à l'étape a) sont sélectionnés dans le groupe consistant en de l'amidon, de l'amidon réticulé, du ficoll, du polysucrose, de l'alcool polyvinylique, de la gélatine, de l'hydroxyméthyl cellulose, de l'hydroxéthyl cellulose, de l'hydroxypropyl cellulose, de l'hydroxypropyl-éthyl cellulose, de l'hydroxypropylméthyl cellulose, de la sodium carboxyméthyl cellulose, de l'acétate de cellulose, un sodium alginate, des esters d'anhydride polymaléïque, des polyortho esters, une polyéthylèneimine, un polyéthylène glycol, un méthoxypolyéthylène glycol, un éthoxypolyéthylène glycol, un polyoxyéthyléne oxyde, un anhydride de poly(1,3 bis(p-carboxyphénoxy)propane-co-sébacique, un N,N-diéthylaminoacétate, des copolymères blocs de polyoxyéthylène et de polyoxypropylène.

15. Procédé selon la revendication 13 dans lequel le rapport en poids des deux polymères utilisés à l'étape a) est dans l'intervalle de 20:80 à 80:20.

16. Procédé selon la revendication 15 dans lequel la matrice polymérique est sélectionnée parmi l'amidon et le ficoll, l'amidon et le polysucrose, l'amidon et l'alcool polyvinylique, l'amidon et la gélatine, l'amidon et l'hydroxyéthyl cellulose, l'hydroxyéthyl cellulose et l'hydroxypropyl cellulose, la gélatine et l'hydroxyéthyl cellulose, la gélatine et l'alcool polyvinylique, le polysucrose et l'alcool polyvinylique, et la sodium carboxyméthyl cellulose et l'alginate de sodium.

17. Procédé selon la revendication 16 dans lequel la matrice polymérique comprend l'amidon et le ficoll et le rapport en poids de l'amidon au ficoll est de 85:15 à 60:40.

18. Procédé selon la revendication 17 dans lequel le rapport en poids est de 75:25 à 65:35.

19. Procédé selon la revendication 16 dans lequel la matrice polymérique comprend l'amidon et l'alcool polyvinylique et le rapport en poids de l'amidon à l'alcool polyvinylique est dans l'intervalle de 35:65 à 65:35.

20. Procédé selon la revendication 19 dans lequel le rapport en poids est de 40:60 à 60:40.

21. Procédé selon la revendication 16 dans lequel la matrice polymérique comprend du polysucrose et de l'alcool polyvinylique et le rapport en poids du polysucrose à l'alcool polyvinylique est de 35:65 à 65:35.

22. Procédé selon la revendication 16 dans lequel la matrice polymérique comprend du ficoll et une cellulose sélectionnée dans le groupe consistant en hydroxyméthyl cellulose, hydroxyéthyl cellulose, l'hydroxypropyl cellulose, l'hydroxypropyl-éthyl cellulose, l'hydroxypropyl-méthyl cellulose, la sodium carboxyméthyl cellulose et l'acétate de cellulose et le rapport en poids de la cellulose au ficoll est dans l'intervalle de 80:20 à 65:35.

23. Procédé selon la revendication 13 ou 14 dans lequel l'ingrédient actif est sélectionné parmi la somatropine bovine, les oestrogènes, les androgènes, l'insuline, les facteurs de croissance d'insuline, l'interleukine-I, l'interleukine-II et les cytokines.

24. Procédé selon la revendication 13 ou 14 dans lequel l'ingrédient actif est sélectionné parmi le vaccin MMR (rougeole, oreillons et rubéole), le vaccin de la typhoïde, le vaccin de l'hépatite A, le vaccin de l'hépatite B, le virus de l'herpès simplex, les toxoïdes bactériens, la sous-unité-B de la toxine du choléra, le virus du vaccin de la grippe, le virus du vaccin de la coqueluche, le virus de la vaccine, un adénovirus, la variole du canari, le virus du vaccin de la polio, le virus du vaccin de la malaria, le bacillus calmette geurin, le klebsiella pneumoniae, et des glycoprotéines d'enveloppe de VIH.

25. Méthode pour augmenter la production de lait d'une vache comprenant l'administration en tant qu'hormone dans une formulation à libération contrôlée selon la revendication 1 ou la revendication 2.
